Europäisches Patentamt

European Patent Office

Office européen des brevets

(11) Publication number: **0 222 489**
**A1**

## (12) EUROPEAN PATENT APPLICATION

(21) Application number: 86307581.8

(22) Date of filing: 01.10.86

(51) Int. Cl.⁴ **C07C 19/00** , **C07C 17/42** , **B41M 5/12**

(30) Priority: 01.10.85 DE 3534984

(43) Date of publication of application:
20.05.87 Bulletin 87/21

(84) Designated Contracting States:
**BE DE ES FR GB IT NL SE**

(71) Applicant: **IMPERIAL CHEMICAL INDUSTRIES PLC**
**Imperial Chemical House Millbank**
**London SW1P 3JF(GB)**

(72) Inventor: **Bieding, Robert**
**Drachenfelsstrasse 14**
**D-5463 Unkel(DE)**
Inventor: **Nestler, Heinz**
**Rembrandtstrasse 73**
**D-5210 Troisdorf(DE)**

(74) Representative: **Oldroyd, Alan et al**
**Imperial Chemical Industries PLC Legal**
**Department Patents PO Box 6 Bessemer**
**Road**
**Welwyn Garden City Herts, AL7 1HD(GB)**

(54) Stabilised chloroparaffins.

(57) The invention concerns the stabilisation of chloroparaffins to produce stabilised chloroparaffins which are suitable for use as solvents in self-copy papers in which they no longer lead to discolouration of the leuco-base or to appreciable fogging in the paper. The stabilised chloroparaffins contain magnesium alcoholates in quantities between 0.05 and 2.0% by weight based on the chloroparaffin. The magnesium alcoholates can be derived from aliphatic alkanols or from ether alcohols; the preferred stabiliser is magnesium methylglycolate.

## STABILISED CHLOROPARAFFINS

The present invention relates to stabilised chloroparaffins which are particularly suitable for use as solvents in self-copy papers or pressure-sensitive copying papers, without the occurrence of appreciable fogging or discolouration of the papers through decomposition of the chloroparaffins.

Chloroparaffins are used extensively as solvents, plasticisers, diluents or flame-retarding additives in many industrial fields of use, e.g. in paints and varnishes or in the mineral oil and plastics industries. Since aliphatically bound chlorine is readily cleaved off through the action of light, heat and chemical reactions, practically all commercially available chloroparaffins are given a basic stabilisation in order to inhibit or to retard the decomposition. This basic stabilisation includes inter alia epoxides, for example epoxidised oils or resins and anti-oxidants, for example sterically hindered phenols. For most fields of use, chloroparaffins stabilised in this way are well suited.

It has been found, however, that in the use of chloroparaffins as solvents for leucodyes which, enclosed in microcapsules, are used in self-copy papers, the stabilising mixtures used until now are insufficient. In this self-copy paper technology, which is described in detail in the "Wochenblatt fur Papierfabrikation - [Papermaking Weekly] 16 (1980) pp 607-612 and 21 (1982) pp 767-776", chloroparaffins act as solvents for leucodyestuffs, which are embedded in microcapsules. These microcapsules are coated onto the top paper sheet. When pressure is applied to the top sheet, the microcapsules break and release the leucobase which acts as a colour-former and reacts with a reactive acceptor layer coated onto the lower sheet to produce a coloured mark. The acceptor layer contains for example organic resins, such as phenolformaldehyde condensation products, or reactive inorganic pigments (e.g. reactive clays of the montmorillonite or attapulgite type after acid treatment).

When chloroparaffins stabilised with the known stabiliser mixtures are used as solvents in this field of use, a discolouration often takes place spontaneously when the leucodye is dissolved in the chloroparaffin, particularly after a heat treatment; or else, after the microencapsulation and inclusion into the capsule layer of the top sheet an undesirable colour fogging gradually forms on the paper surface on exposure to light and air.

In order to avoid these disadvantages, it has been proposed in DE-AS 20 37 159, that a chloroparaffin be used whose carbon chain contained not more than 18 C-atoms, and whose chlorine content is below 60%. In addition, the chloroparaffins should have no branched chains. The use of such compounds presupposes special production of these compounds. Further, in DE-PS 27 02 017, it was proposed that alkaline earth sulphonates and/or phenates and/or nitrogen-containing derivatives of an alkylsuccinic acid should be added. These compounds display particularly good effects when they are used in combination, so that it is always necessary to use several compounds in order to achieve a good effect.

The problem was therefore to stabilise chloroparaffins in such a way that they do not attack leucobases that are used in self-copy papers, so that the papers do not discolour prematurely. The desired stabiliser should display stabilising activity even in chloroparaffins having a high degree of chlorination and a long carbon chain. Furthermore, it should also have this effect even for branched-chain chloroparaffins.

According to the present invention, stabilised chloroparaffins have now been found which are characterised in that they contain magnesium alcoholates.

In general the magnesium alcoholate is added as additional stabiliser to a chloroparaffin which already contains known stabilisers. In this case, amounts of between 0.05 and 2 wt %, based on the chloroparaffin, suffice to achieve an adequate stabilisation with respect to the leucodyes.

In cases where the magnesium alcoholate is insufficiently soluble in the chloroparaffin, it is desirable to use a solution aid. Suitable for this purpose are for example ether alcohols, polyethylene glycols, or polyglycol ethers. Preferred are ether alcohols, for example methoxypropanol.

As magnesium alcoholates can be used either those whose alcohol groups are derived from simple straight-chain or branched-chain aliphatic alkanols whose carbon chains possess 1 to 8, preferably 2 to 4 C-atoms, or those whose alcohol grouping has one or several ether oxygen atoms, for example the methyl-or ethyl-(poly)ethylene glycols. Examples of magnesium alcoholates that can be used include magnesium methoxide, magnesium isopropoxide and magnesium methylglycolate. The preferred stabiliser is magnesium methylglycolate.

The chloroparaffins to be stabilised are generally mixtures because of the way in which they are produced. They can have a chain length of 10 to 35 C-atoms, and a chlorine content between 10 and 71% by weight. The carbon chains of such chloroparaffins are usually straight but branched chains can be present in proportions of up to about 10%.

In general, the stabilisers according to the invention are added to chloroparaffins that have already been prestabilised, or are used together with known stabilising agents. The stabilisers are compatible with these known stabilisers and have no practical effect on their action. Leucobases that are used in self-copy papers dissolve well in the chloroparaffins stabilised according to the invention. Warming may perhaps be desirable or even necessary. The solution obtained retains its original shade even on heating for 45 minutes at 120°C. Even emulsification of such a leucobase solution with water and prolonged standing of the chloroparaffin emulsion does not result in premature colour formation.

The invention is illustrated by the following Examples in which all percentages are by weight.

EXAMPLE I

In each case, 40 g of a chlorinated, essentially straight-chained paraffin with the chain length stated in Table I and the correspondong chlorine content stated there, was mixed with I% Crystal Violet lactone - (commercial product Reakt-Violett 626 from BASF AG) and heated for I5 minutes at 80°C. Then the mixture was cooled to room temperature and the optical density of the mixture was determined at 594 nm in a 0.5 cm glass cell by means of a spectrophotometer.

The results are shown in Table I; they show that the use of the stabilisers according to the invention produces considerable inhibition of colour formation during dissolution of a leucodye in chloroparaffins.

## TABLE 1

| Paraffin chain length | Cl content | Additives | Optical density |
|---|---|---|---|
| $C_{14} - C_{17}$ | 45% | 1% epoxidised resin | 0.26 |
| $C_{14} - C_{17}$ | 45% | 1% epoxidised resin<br>0.5% Mg methylglycolate | 0.04 |
| $C_{14} - C_{17}$ | 20% | 0.5% epoxidised resin | 0.25 |
| $C_{14} - C_{17}$ | 20% | 0.5% epoxidised resin<br>0.5% Mg methylglycolate | 0.09 |
| $C_{10} - C_{13}$ | 61% | 1% epoxidised resin | 0.37 |
| $C_{10} - C_{13}$ | 61% | 1% epoxidised resin<br>0.5% Mg ethoxide | 0.10 |
| $C_{20} - C_{28}$ | 30% | 0.5% epoxidised resin | 0.22 |
| $C_{20} - C_{28}$ | 30% | 0.5% epoxidised resin<br>0.5% Mg isopropoxide | 0.08 |

## EXAMPLE 2

Since during the production of self-copy papers, temperatures of l00°C occur at times, the thermal stability of the solvent used is of great importance. If the stability of the solvent is only slight, this results in colour reactions in the microcapsules, and hence premature discolouration (fogging) of the self-copy papers.

In order to confirm the stabilising effect of the stabilisers according to the invention at high temperatures, the procedure of Example I was used to prepare solutions of the leucodye specified in Example I in various chloroparaffins, and these were then heated for 30 or 45 minutes at l20°C. After cooling of the samples, the optical densities were measured as in Example I. The results obtained are shown in Table 2.

### TABLE 2

| Paraffin chain length | Cl content | Additives | Optical density after | | |
|---|---|---|---|---|---|
| | | | 0 min | 30 min | 45 min |
| $C_{14} - C_{17}$ | 45% | 1% epoxidised resin | 0.264 | 0.392 | 0.474 |
| $C_{14} - C_{17}$ | 45% | 1% epoxidised resin 0.5% Mg methyl-glycolate | 0.041 | 0.046 | 0.047 |
| $C_{10} - C_{13}$ | 49% | 1% epoxidised resin 0.5% Mg methyl-glycolate | 0.062 | 0.075 | 0.075 |

## EXAMPLE 3

The water tolerance of the solution consisting of solvent and leucodye, prepared by the procedure of Example I, provides further evidence for the effectiveness of the additives according to the invention. 40 g of these solutions were mixed with 40 g distilled water using a fast-running basket stirrer with heating at 50°C.

After separation of the phases, the water and the water-chloroparaffin mixture were checked for discolouration.

The result was that dye solutions prepared using chloroparaffins stabilised only with epoxidised resins displayed a strong discolouration both of the aqueous phase and of the water-chloroparaffin mixture. However, all formulations with chloroparaffins as solvent and stabilised according to the invention showed no discolouration of the individual phases after the water treatment.

## Claims

1. Stabilised chloroparaffins with a chain length of 10 to 35 C-atoms and a chlorine content between 10 and 71%, characterised in that the stabilised chloroparaffins contain magnesium alcoholates in proportions between 0.05 and 2% by weight based on the chloroparaffins.

2. Stabilised chloroparaffins according to Claim 1, characterised in that they contain magnesium methylglycolate as the magnesium alcoholate.

3. Stabilised chloroparaffins according to Claim 1 or 2 which also contain one or more conventional stabilisers.

4. A self-copy paper having applied thereon a coating composition comprising a solution of a leucobase in a stabilised chloroparaffin as claimed in Claim 1, 2 or 3.

5. A self-copy paper as claimed in Claim 4 wherein the solution of the leucobase in the stabilised chloroparaffin is formed with the aid of a solution aid.

6. A self-copy paper as claimed in Claim 5 wherein the solution aid is an ether alcohol.

7. A self-copy paper as claimed in Claim 6 wherein the solution aid is methoxypropanol.

8. Microcapsules for use in self-copy paper comprising encapsulated droplets of a solution of a leucobase in a stabilised chloroparaffin as claimed in Claim 1, 2 or 3.

9. Use of stabilised chloroparaffins according to Claim 1, 2 or 3 as a solvent for leucobases which are used in self-copy papers.

10. Use of stabilised chloroparaffins as a solvent according to Claim 9 in combination with an ether alcohol as a solution aid.

European Patent
Office

**EUROPEAN SEARCH REPORT**

Application number

EP 86 30 7581

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-1 935 744 (PRODUITS CHIMIQUES PECHINEY-SAINT-GOBAIN) * Page 2 * | 1,3 | C 07 C 19/00 C 07 C 17/42 B 41 M 5/12 |
| D,X | DE-A-2 037 159 (FUJI PHOTO FILM CO.) * Claims * | 4,8 | |
| D,X | DE-A-2 702 017 (I.C.I.) * Claims * | 4,8-10 | |

TECHNICAL FIELDS SEARCHED (Int. Cl.4)

C 07 C 17/00
B 41 M 5/00

The present search report has been drawn up for all claims

| Place of search | Date of completion of the search | Examiner |
|---|---|---|
| THE HAGUE | 12-01-1987 | VAN GEYT J.J.A. |

EPO Form 1503 03 82

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document